Europäisches Patentamt

(19) European Patent Office

Office européen des brevets

(11) Publication number: **0 073 593**
A1

(12) **EUROPEAN PATENT APPLICATION**

(21) Application number: **82304318.7**

(22) Date of filing: **16.08.82**

(51) Int. Cl.³: **G 01 N 33/54,** G 01 N 33/58

(30) Priority: **01.09.81 US 298426**
**28.10.81 US 315920**

(43) Date of publication of application: **09.03.83**
**Bulletin 83/10**

(84) Designated Contracting States: **CH DE GB LI**

(71) Applicant: **E.I. DU PONT DE NEMOURS AND COMPANY,**
**Legal Department 1007 Market Street, Wilmington**
**Delaware 19898 (US)**

(72) Inventor: **Chang, Shung-Ho, 6 Old Gate Lane,**
**Wilmington Delaware 19810 (US)**

(74) Representative: **Collier, Jeremy Austin Grey et al,**
**J.A.Kemp & Co. 14, South Square Gray's Inn, London**
**WC1R 5EU (GB)**

(54) **Size-exclusion heterogeneous immunoassay.**

(57) A novel non-radioisotopic heterogeneous immunoassay is provided in which size-exclusion chromatography is utilized to separate the products of a competitive binding reaction. The amount of the label in the antigen-antibody complex is inversely proportional to the amount of analyte in a biological fluid.

EP 0 073 593 A1

ACTORUM AG

## TITLE
SIZE-EXCLUSION HETEROGENEOUS IMMUNOASSAY
## DESCRIPTION
### Technical Field

This invention relates to a novel immunoassay method employing a size-exclusion separation step for the measurement of haptens of interest in biological fluids.

### Background Art

Radioimmunoassays (RIAs) are widely used in the assay of haptens such as hormones or drugs. In these assays, a known quantity of radiolabelled hapten, the sample containing hapten (analyte), and the antibody to the hapten are mixed. Following an incubation period which allows for the competitive binding reaction to occur, the free and antibody-bound hapten are separated. The amount of radioactivity in the free or bound fractions is measured to indicate the concentration of analyte in the biological sample.

Separation steps employed in RIA include fractional precipitation with ammonium sulfate or polyethylene glycol, adsorption to dextran-coated charcoal, immunological precipitation with a second antibody, or solid-phase separation using analyte-coated tubes or analyte covalently attached to beads. (These are described, for example, in "Enzymes as Immunochemical Labels" by Edward T. Maggio in Enzyme-Immunoassay, ed. Edward T. Maggio, CRC Press, Inc., Boca Raton, FL, 1980, pages 53-70). These techniques can be laborious to perform, difficult to automate, and often require specialized reagents for each particular immunoassay procedure.

A variety of separation techniques has also been applied in enzyme linked immuno-sorbent assays

2      0073593

(ELISA); see, for example, United States 3,654,090, issued April 4, 1972 to Schuurs, et al., and in other types of heterogeneous immunoassays. The solid-phase separation and immunological precipitation techniques are the separation techniques most commonly used.

Size exclusion is well known in biochemistry, clinical chemistry, and immunology but its use as a separation technique in a heterogeneous immunoassay has been limited to an RIA for estrogens in serum (Jens Larsen, et al., "Methods in Enzymology," Volume 70, Part A, ed. H. Van Vunakis and J. J. Langone, Academic Press, New York, NY, 1980, pages 315-322). However, radioimmunoassays have several disadvantages including reagent instability, the need for expensive detection instruments, and the safety hazards involved in handling, storing, and disposing of the radioactive material. There is a need for a separation technique which utilizes a multipurpose column useful for the separation of a variety of analytes from their respective antigen-antibody complexes and for different immunoassay technologies. There is a further need for a separation technique which is adaptable to automation.

DISCLOSURE OF THE INVENTION

The heterogeneous immunoassay method of this invention for the measurement of haptens in biological fluids comprises the steps of:

(A) mixing the fluid suspected of containing the hapten, a nonradioisotopically labelled hapten and an antibody to the hapten;

(B) incubating the materials mixed in step A to allow the competitive binding reactions to proceed;

(C) passing the incubated mixture from step B through a gel filtration column packed with a material having a fractionation range suitable for the separation of the labelled hapten-antibody complex from the labelled hapten; and

(D) measuring the amount of the labelled hapten-antibody complex eluted from the column.

## DESCRIPTION OF THE INVENTION

In heterogeneous immunoassays, a labelled hapten, a sample containing the hapten (analyte) and an antibody to the hapten are incubated together for the competitive binding reaction to proceed. A separation step is then necessary to separate the hapten and labelled hapten from antigen-antibody complexes. The term antigen includes both hapten and labelled hapten. The amount of labelled hapten can then be measured, often in the bound (complex) fraction, to indicate the concentration of analyte in the sample.

The immunoassay method of this invention, utilizing size-exclusion separation, offers a technique which provides the separation for all heterogeneous immunoassay techniques where a large molecular size difference exists between the hapten and labelled hapten, on the one hand, and the antigen-antibody complex fractions. This separation technique is applicable to ELISA, heterogeneous fluorescence immunoassay, heterogeneous immunoassays based upon cofactor labelled hapten (United States 4,238,565, issued December 9, 1980 to W. E. Hornby, et al.), and enzyme inhibitor labelled hapten (United States 4,273,866, issued June 16, 1981 to Houston F. Voss, et al.).

A major advantage of this invention is its universal applicability in these four types of heterogeneous immunoassays. Furthermore, the same size-exclusion separation column can be utilized in all of these immunoassays. This feature is especially important in facilitating the automation of heterogeneous immunoassays for clinically important analytes such as hormones or drugs.

A variety of column materials can be used for the separation of materials on the basis of molecular weight. The cross-linked dextrans (available from Pharmacia Fine Chemicals AB, Uppsala, Sweden, as the Sephadex®, Sephacryl® or Sepharose® series), polyacrylamide gels (available from Bio-Rad Laboratories, Richmond, CA, as the Bio-Gel P series) and agarose gels (also available from Bio-Rad Laboratories as the Bio-Gel A series) are commercially available. Controlled pore-size glass beads (CPG), coated with glycerol propylsilane [see, for example, Regnier, et al., J. Chromatographic Sci., Volume 14, 316 (1976)], can also be used as column materials for size-exclusion separation.

The choice of column material for a particular application depends upon various factors such as exclusion limits, elution speed, mechanical strength of the column material, back pressure generated during the separation, ease of packing, and resolution capability.

Many of the available column materials lack the mechanical strength to withstand the pressures exerted during the column packing and elution steps. In applications where the elution is forced, the presence of back pressure can result in leaks if the columns are not made for high pressure. These problems can be avoided by choosing crosslinked column materials especially prepared for mechanical strength and of medium particle size for elimination of back pressure problems. It is also important to process the column materials by removing the fine particle fragments and to properly pack the columns.

In gel filtration chromatography, molecules larger than the largest pores of the particles of the column material, that is, above the exclusion limit, cannot penetrate into the pores of the particles and are eluted first at the void volume as they pass through the column in the liquid phase outside of the particles. Smaller molecules can penetrate the particles to varying extent depending upon their size and shape and are eluted in order of decreasing molecular size.

Size-exclusion chromatography is a special case of gel filtration chromatography in which the column material is so chosen that its exclusion limit is not above the molecular weight of the fraction desired to be eluted at the void volume. A converse requirement of not having undesired fractions eluted at the void volume is that these undesired fractions have molecular weights below the exclusion limit.

Chromatographic separation is greatly dependent, among other factors, on column size. Small chromatographic columns are desirable for use in automated clinical systems where reproducibility, ease of manufacture, speed of elution, and cost are primary concerns. Total separation, however, is difficult in these cases since there is little liquid volume eluted between the void volume and the total bed volume. To achieve successful separation of different size molecules, the choice of a column material with the proper fractionation range is very important. Table 1 indicates the fractionation range of some of the available column materials.

### Table 1

### Chromatographic Properties
### of Various Column Materials

| Column Materials | Fractionation Ranges (Daltons) |
|---|---|
| Sepharose 4B | 60,000 – 20,000,000 |
| Sepharose 6B | 10,000 – 4,000,000 |
| Bio-Gel A-5 m | 10,000 – 5,000,000 |
| Bio-Gel A 1.5 m | 5,000 – 1,500,000 |
| Bio-Gel A-0.5 m | 5,000 – 500,000 |
| Sephadex G-50 | 1,500 – 30,000 |
| Bio-Gel P-4 | 800 – 4,000 |
| Bio-Gel P-6 | 1,000 – 6,000 |
| Bio-Gel P-10 | 1,500 – 20,000 |
| Sephacryl S-200 | 5,000 – 250,000 |

The antigen-antibody complexes formed from the reaction of a hapten (the analyte) with an IgG antibody have molecular weights of approximately 160,000 daltons. In contrast, both the analytes, such as hormone or drug, and the labelled analyte (conjugate), which is a hapten covalently attached to a label such as fluorophore, cofactor or inhibitor, are typically small molecules of molecular weight less than 3,000 daltons. Thus, the separation of these two different molecular weight species is practical with most of the column materials listed in Table 1.

In the case of enzyme-labelled hapten, the molecular weight of the conjugate will primarily be determined by the size of the enzyme. Therefore, size-exclusion separations of relatively large enzymes (molecular weights greater than 5,000 daltons) are more difficult with many of the clinically desirable, small column systems than with systems employing large columns. For example, the separation of a hapten labelled with horseradish peroxidase (molecular weight approximately 40,000 daltons) from the hapten-antibody complex (molecular weight approximately 200,000 daltons) is relatively difficult using small columns.

The use of low molecular weight enzymes, however, enhances the applicability of size-exclusion separation to the clinically-useful enzyme immunoassays of this invention. Microperoxidase is a small enzyme of molecular weight approximately 2,000 daltons. Conjugates of hapten and microperoxidase can readily be separated from the antigen-antibody complexes using the small columns with appropriate packing material.

Following the separation of the antigen-antibody complexes from the hapten and labelled hapten, the measurement of the quantity of the label in the complex can be carried out by known methods depending on the type of label utilized.

The examples which follow are illustrative of the invention.

<div align="center">

EXAMPLE 1

Size Exclusion Fluorescence
Immunoassay for Gentamicin

</div>

A.   Preparation of Column

The column material, Bio-Gel P-10 resin, is washed extensively with distilled water and suspended in 0.1 M phosphate buffer, pH 7.2. The column material is used in columns designed for use in an automatic clinical analyzer (available from E. I. du Pont de Nemours and Company as the 'aca'). The column is a plastic tube approximately 5.5 mm in diameter and 88 mm long with rubber stoppers at both ends which allow for automatic sample and diluent entry and eluent exit into an analytical test pack. Such an analytical test pack is described in Re 29,725, issued August 8, 1978 to D. R. Johnson, et al. which is hereby incorporated by reference.

B.    Preparation of Gentamicin-Fluorescein Conjugate

The gentamicin-fluorescein conjugate is synthesized by a procedure similar to that described by Watson, et al., Clin. Chem. Acta, Volume 73, 51 (1976). 6.82 mg of gentamicin sulfate (available from Sigma Chemical Co., 62% active ingredient) is reacted with 5.0 mg of fluorescein isothiocyanate in 10 ml of 0.05 M carbonate buffer, pH 9.0, for two hours at room temperature. 4 ml of the resultant mixture is applied to a Sephadex G-15 column (1.5 x 97 cm) and is eluted with the carbonate buffer at a rate of approximately 0.5 ml/min with a peristaltic pump. The unreacted gentamicin sulfate is eluted without retardation and the immunoreactive gentamicin-fluorescein conjugate is found in the first fluorescent peak. The fractions containing this first fluorescent peak are pooled. Small aliquots of the pool are frozen until ready for use. The conjugate is tested for immunoreactivity with antigentamicin antibodies by chromatographically separating the free and antibody-bound fluorescence and it is found to be greater than 90% pure.

C.    Assay Protocol for Serum Gentamicin

Gentamicin sulfate standards are prepared by dissolving gentamicin sulfate in water and making dilutions with normal human serum to the appropriate concentrations. 0.050 ml of the human serum containing gentamicin (prediluted 10-fold with 0.1 M sodium phosphate buffer, pH 7.2) is added to 0.050 ml of antigentamicin (rabbit antisera from Miles Laboratories, Inc., prediluted 30-fold with the same phosphate buffer). After a 1-min incubation period at room temperature, 0.050 ml of the gentamicin-fluorescein conjugate (0.001 mg/ml in the phosphate buffer) is added. After a further 1-min incubation period at room temperature, the mixture is transferred to an 'aca' microsample cup. The 'aca' automatically performs the addition of the sample to the column and its elution. The sample volume is 0.100 ml, the elution volume is 0.900 ml, and the elution speed is 0.50 ml/min. After the elution is complete, 4.0 ml of the phosphate buffer is added for a total of 5.0 ml volume in the 'aca' test pack. The contents of the 'aca' pack are transferred to a cuvette for the measurement of the fluorescence in a spectrofluorimeter (Aminco Model SPT-500). The level of fluorescence observed is a measure of the gentamicin-fluorescein-antibody complex which is eluted directly through the column. This level is inversely proportional to the quantity of gentamicin in the sample and is given in Table 2. The assay is reproducible (coefficient of variation less than 3% at 3.0 µg/ml). The cross-reactivity of the assay is tested with 1,000 µg/ml of several closely related antibiotics. The results, shown in Table 3, indicate that the cross-reactivity is low and, in Table 4, show that the recovery of gentamicin is good even in the presence of other antibiotics. The data in Table 2 when compared to those of Tables 3 and 4 show some differences in relative fluorescence values due to day-to-day instrument variations.

## Table 2

### Standard Curve for
### Gentamicin Fluorescence Immunoassay

| Gentamicin µg/ml | Relative Fluorescence |
|---|---|
| 0 | 139 |
| 0.5 | 130 |
| 1.0 | 120 |
| 3.0 | 85 |
| 5.0 | 72 |
| 7.5 | 59 |
| 10.0 | 50 |
| 15.0 | 37 |
| 20.0 | 32 |

## Table 3

### Cross-Reactivities in
### Gentamicin Fluorescence Immunoassay

| Sample | Relative Fluorescence | Gentamicin Concentration (µg/ml) | Cross-Reactivity (%) |
|---|---|---|---|
| 1000 µg/ml Tobramycin | 114 | 0.1 | <0.1 |
| 1000 µg/ml Streptomycin | 117 | 0.2 | <0.1 |
| 1000 µg/ml Neomycin | 117 | 0.1 | <0.1 |
| 1000 µg/ml Kanamycin | 118 | 0 | <0.1 |
| Water Control | 118 | 0 | 0 |

## Table 4
### Recovery of Gentamicin in
### Gentamicin Fluorescence Immunoassay

| Antibiotic in Sample Containing 3.0 µg/ml Gentamicin | Relative Fluorescence | Gentamicin Concentration (µg/ml) | Recovery (%) |
|---|---|---|---|
| 1000 µg/ml Tobramycin | 65 | 3.0 | 100 |
| 1000 µg/ml Streptomycin | 70 | 2.8 | 95 |
| 1000 µg/ml Neomycin | 66 | 3.0 | 100 |
| 1000 µg/ml Kanamycin | 66 | 3.0 | 100 |
| Water Control | 66 | 3.0 | 100 |

## EXAMPLE 2
### Size Exclusion Fluorescence
### Immunoassay for Tobramycin

A.   Preparation of Column

A column identical to that used in Example 1 is utilized in this Example.

B.   Preparation of Tobramycin-Fluorescein Conjugate

The procedure of Example 1B is used except that 0.8 mg of tobramycin (available from Eli Lilly & Co. as Nebian, 60% active ingredient) is used instead of the gentamicin sulfate.

C.   Assay Protocol for Serum Tobramycin

The procedures utilized in Example 1 are followed except that the sample is prediluted 20-fold with the phosphate buffer and the antibody (antisera from Atlantic Antibodies, Inc.) is prediluted 30-fold with the phosphate buffer. A standard curve is given in Table 5 for the tobramycin assay. Studies show little cross-reactivity with gentamicin, neomycin, and streptomycin but kanamycin shows significant cross-reactivity (approximately 20%), see Table 6.

## Table 5

### Standard Curve for
### Tobramycin Fluorescence Immunoassay

| Tobramycin µg/ml | Relative Fluorescence |
|---|---|
| 0 | 123 |
| 0.5 | 108 |
| 1.0 | 104 |
| 3.0 | 66 |
| 5.0 | 52 |
| 7.5 | 45 |
| 10.0 | 37 |
| 15.0 | 29 |
| 20.0 | 20 |

## Table 6

### Cross-Reactivities in
### Tobramycin Fluorescence Immunoassay

| Sample | Relative Fluorescence | Tobramycin Concentration (µg/ml) | Cross-Reactivity (%) |
|---|---|---|---|
| 100 µg/ml Gentamicin | 102 | 1.0 | 1.0 |
| 100 µg/ml Neomycin | 118 | 0.3 | 0.3 |
| 100 µg/ml Kanamycin | 21 | 20.0 | 20.0 |
| 100 µg/ml Streptomycin | 102 | 1.0 | 1.0 |

## EXAMPLE 3

### Size Exclusion Cofactor Immunoassay for Theophylline

The reagents for this immunoassay are described in U.S. 4,238,565, issued December 9, 1980, which is hereby incorporated by reference. The measurement of theophylline is carried out by the 'aca' instrument after separation of the FAD-theophylline conjugate from the antibody-conjugate complex.

A.  A 0.050-ml quantity of serum containing theophylline is mixed with 0.050 ml of antibody to theophylline.  After a 1-min incubation period, 0.050 ml of FAD-theophylline conjugate (5.6 nM/ml) is added.  After a further incubation period of 1 min, the mixture is transferred to an 'aca' sample cup.  A 0.100-ml portion of the mixture from the sample cup is transferred onto a column prepared as in Example I(A).  0.900 ml of buffer (0.1 M phosphate, pH 7.2) is used to elute the antibody-conjugate complex into the test pack at a rate of 0.5 ml/min.  Following elution, 4.0 ml of the phosphate buffer is added for a total of 5.0 ml in the analytical test pack.

B.  The analytical test pack utilized for the cofactor theophylline immunoassay contains 0.070 ml of 10 mg/ml theophylline in water in dimples 1 and 2, 0.050 ml of glucose oxidase apoenzyme (diluted to 5 nM/ml with 30% glycerol/water solution and an equal volume of antiglucose oxidase antiserum) in dimples 3 and 4, 0.050 ml of 4-aminoantipyrene (3.8 mg/ml) and horseradish peroxidase (5.2 mg/ml) in dimple 5, $\beta$-D-glucose (90 mg in tablet form also containing 5 mg polyethylene glycol 6000) in dimple 6, and 0.050 ml of dihydroxybenzene sulfonic acid (33.65 mg/ml) in dimple 7.  The reagents in dimples 1 through 4 are delivered into the test pack at the first reagent addition step followed after 3.5 min at 37°C by the reagents in dimples 5 through 7.

The amount of FAD-theophylline conjugate-antibody coupled eluted into the test pack in step A is measured after freeing the conjugate from the complex by the excess theophylline added during the first reagent addition step.  The amount of glucose oxidase formed from the conjugate and apoglucose oxidase is measured in a coupled assay with horseradish peroxidase in a rate measurement using a 510 nm filter. The level of observed enzyme activity is a measure of the FAD-theophylline conjugate-antibody complex which is eluted from the column. This level is inversely proportional to the quantity of theophylline in the sample and is given in Table 7.

## Table 7

### Standard Curve for Theophylline Cofactor Immunoassay

| Theophylline (μg/ml) | Rate ($\Delta A$/min at 510 nm) |
|---|---|
| 0 | 1.036 |
| 1 | .964 |
| 5 | .976 |
| 20 | .733 |
| 30 | .509 |

CLAIMS

1.  A heterogeneous immunoassay method for the measurement of haptens in biological fluids comprising the steps of:

(A)  mixing said fluid suspected of containing said hapten, a nonradioisotopically labelled hapten and an antibody to the hapten;

(B)  incubating the materials mixed in step A to allow the competitive binding reactions to proceed;

(C)  passing the incubated mixture from step B through a gel filtration column packed with a material having a fractionation range suitable for the separation of the labelled hapten-antibody complex from the labelled hapten; and

(D)  measuring the amount of the labelled hapten-antibody complex eluted from the column.

2.  The heterogeneous immunoassay of Claim 1 wherein the hapten is selected from the group consisting of drug, hormone, vitamin and steroid.

3.  The heterogeneous immunoassay of Claim 1 wherein the label in the labelled hapten is selected from the group consisting of enzyme, cofactor and fluorophore.

4.  A heterogeneous immunoassay of Claim 1 wherein the column material in a size-exclusion column has an exclusion limit not above the molecular weight of the labelled hapten-antibody complex but above the molecular weight of the labelled hapten.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | G 01 N   33/54 |
| Y | BIOLOGICAL ABSTRACTS, vol. 73, no. 4, 1982, page 2463, col. 1, no. 23952, Philadelphia (USA); T.P.DEHENY   et   al.:   "Gel filtration separation of bound and free antigens in radioimmunoassay of prostaglandin F2alpha".   &   PROSTAGLANDINS, 21(6):   1003-1014,   1981.   *The whole abstract* | 1,2,4 | G 01 N   33/58 |
| Y | US-A-4 104 026   (G.BROOKER et al.) *Column 1, lines 39-61; column 4, line 5 - column 9, line 65; column 13, lines 29-56; column 13, lines 29-56; table III; claims 1-14* | 1-4 | |
| | --- | | |
| Y | EP-A-0 022 005   (INSTITUT NATIONAL DE LA SANTE ET DE LA RECHERCHE MEDICALE) *The whole document* | 1-4 | **TECHNICAL FIELDS SEARCHED (Int. Cl. ³)**  G 01 N |
| A | US-A-3 442 819   (V.HERBERT) | | |
| A | GB-A-2 017 910   (UNION BARBIDE CORPORATION) | | |
| | ----- | | |

The present search report has been drawn up for all claims

| Place of search THE HAGUE | Date of completion of the search 07-12-1982 | Examiner GRIFFITH G. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82